# EUROPEAN PATENT SPECIFICATION

(11) **EP 0 941 230 B1**
(45) Date of publication and mention of the grant of the patent: **12.05.2004**
(21) Application number: 97951929.5
(22) Date of filing: 20.11.1997
(51) Int. Cl.: C07F 9/50, C07F 15/00, B01J 31/24

(54) **1,3-DIPHOSPHINOPROPANE LIGANDS AND TRANSITION METAL COMPLEXES FORMED THEREFROM, THEIR PREPARATION AND USE**
1,3-DIPHENYLPHOSPHINOPROPAN-LIGANDEN UND ENTSPRECHENDE ÜBERGANGSMETALLKOMPLEXE, DEREN HERSTELLUNG UND VERWENDUNG
LIGANDS DE 1,3-DIPHOSPHINOPROPANE ET COMPLEXES DE METAUX DE TRANSITION FORMES A PARTIR DESDITS LIGANDS, LEUR PREPARATION ET UTILISATION

(30) Priority: 21.11.1996 IT FI960272
(43) Date of publication of application: 15.09.1999
(73) Proprietor: CONSIGLIO NAZIONALE DELLE RICERCHE, 00185 Roma (IT)
(72) Inventor: BIANCHINI, Claudio, I-50136 Florence (IT); MELI, Andrea, I-50135 Florence (IT); VIZZA, Francesco, I-50141 Florence (IT)
(74) Representative: Gervasi, Gemma, Dr.
(86) International application number: PCT/EP1997/006493
(87) International publication number: WO 1998/022482

(56) References cited:
- EP-A- 0 311 352
- EP-A- 0 404 228
- EP-A- 0 511 713
- EP-A- 0 571 819
- EP-A- 0 585 493
- EP-A- 0 728 762
- WO-A-91/03262
- DE-A- 3 942 789
- GB-A- 2 242 189
- GB-A- 2 262 284
- Bianchini C. et al: Organometallics, 1995, 14, 54-5459
- Bianchini C. et al: Macromolecules, 1999, 32, 3859-3866

## Description

The invention relates to compounds of general formula (I) 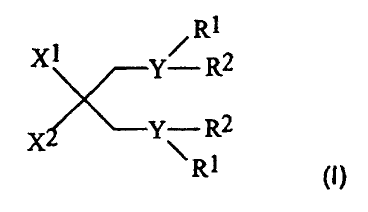 wherein R¹, R² represent hydrocarbyl groups, identical or different from each other, X¹ represents a hydrogen atom, a hydrocarbyl group or a polar group, X² represents a polar group, Y represents phosphorus or arsenic and to their preparation and to their use as ligands in metal complexes; such polar group being chosen among: aryl- or alkyl sulfonic acid, sulfonate alkali salt (-SO₃H, - SO₃⁻M⁺ wherein M⁺ is Li, Na, K) and a quaternary ammonium salt. The invention further relates to coordination compounds that the ligands of formula (I) form with transition metals, to their preparation and to their use as molecular catalysts for reactions in water or in polyphasic systems comprising an aqueous phase, or as heterogeneous catalysts obtained by anchoring their metal complexes to common inorganic and organic supports (silica, alumina, pumice, organic polymers).

### State of the art

It is well-known that many chemical reactions require the presence of a catalyst, generally a metal complex, to take place with high rate and selectivity. For reactions in aqueous media or in polyphasic systems wherein water is the polar phase, it is necessary for the catalyst to be water-soluble.

Among the transition metal complex catalysts involved in a large variety of reactions, the most common are those which contain a metal centre coordinated by mono- or polydentate phosphine ligands with different hydrocarbyl substituents on the P-donor atoms.

A common strategy for achieving the solubility in water of the ligands involves the introduction of a polar group on one or more substituents on the donor atoms (in the case of phosphorus donors, this is generally achieved by sulfonation of aryl substituents.

Ligands and complexes of the types mentioned above and their application to catalysis are described in: Applied Homogeneous Catalysis with Organometallic Compounds, B. Comils and W. A. Herrmann, Eds.; VCH: Weinheim, Germany 1996, Vol, 2, Chapter 3.1, pp. 573-623.

In the water-soluble phosphine ligands of current use in catalysis, the donor atoms are sterically congested and, eventually, electronically deactivated by the polar substituents, however these two effects may decrease the coordination capability of the ligands as compared to similar ligands devoid of the functional group(s) which allow them to be water-soluble. Moreover, the majority of the known water-soluble ligands are unidentate and the selectivities (chemo-, regio- and stereoselectivity) induced by their metal catalysts are generally poor. The stability of these complexes towards ligand dissociation pathways is also rather low. A few water-soluble bidentate ligands are known. In none of them, however, the phosphorus donors are separated by three CH₂ groups and the phosphorus substituents do not bear polar groups.

### Detailed description of the invention

The invention allows one to overcome the problems occasioned by the proximity of the donor atoms and the polar groups by using metal complexes with either 1,3-diphosphinopropane or 1,3-diarsinopropane ligands in which the polar group(s) is (are) bound, directly or by means of an appropriate substituent, to the carbon in the 2-position of the propylenic chain in between the two phosphorus or arsenic atoms. The ligands which are the object of the invention, have the formula (I): 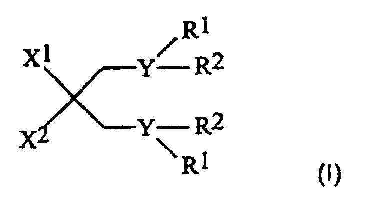 wherein X¹, X², R¹, R² and Y are defined as reported above.

The metal complexes obtainable with these ligands exhibit the following advantages over the known water-soluble ligands: decreased steric hindrance at the donor atoms, which allows the formation of a wider range of stable water-soluble transition metal complexes; increased capability of inducing chemo-, regio- and stereoselective reactions; increased stability of the resulting metal complexes (polydentate effect); possibility of introducing the ligating group (phosphine or arsine) into the ligand framework after the water-soluble molecular structure has already been assembled, and, consequently, possibility of using a wider range of phosphorus or arsenic substituents; possibility of anchoring both ligands and metal complexes on common inorganic and organic supports, and thus eventual application of the supported complexes to heterogeneous catalytic reactions (liquid-solid and gas-solid).

Within the context of the present invention, for hydrocarbyl group we mean: a linear alkyl group, branched or cyclic, containing from 1 to 6 carbon atoms, or an aryl group, generally alkyl-substituted. Preferably, the alkyl group on the phosphorus donor is the cyclohexyl one (-C₆H₁₁), the aryl group is the phenyl one (-C₆H₅), the polar group is *p*-benzylsulphonic or *p*-benzylsulphonate (as alkali salt) (-CH₂(C₆H₄)SO₃H, or -CH₂(C₆H₄)SO₃⁻ Na⁺, K⁺, Li⁺).

The ligands which are the object of the invention can be prepared as exemplified in the procedure shown in the Scheme.

As is apparent from the Scheme, the synthetic strategy for all the diphosphine or diarsine ligands involves as starting compound diethylmalonate or its derivatives obtained by changing the substituent on the central carbon atom. The introduction of the polar group(s) is achieved by ordinary chemical reactions in common organic solvents. In a similar way, the introduction of the donor atoms (P or As) is achieved by reaction between phosphide or arsenide groups and bis-halide compounds already containing the polar group(s).

The ligands obtained in this way are able to coordinate every transition metal in various oxidation states. The complex-formation reactions take place *via* the common methods of coordination chemistry, for example by displacement of labile ligands from their complexes or by reaction between the ligands and inorganic salts of the metals (generally halides).

The following example details the preparation of a 1,3-diphosphinopropane ligand of the formula (NaO₃S(C₆H₄)CH₂)₂C(CH₂PPh₂)₂ [2,2-bis(*p*-Na-sulfonato-benzyl)-1,3-diphenylphosphinopropane] (quoted in the Scheme as la).

### EXAMPLE

### Synthesis of (PhCH₂)₂C(CO₂Et)₂ (2,2-bis(ethylcarboxylate)-1,3-diphenylpropane.

To a solution of PhCH₂CH(CO₂Et)₂ (74.1 g, 0.30 mol) in tetrahydrofuran (THF) (150 mL) at 0 °C under an inert atmosphere (N₂ or Ar) was added portionwise with stirring NaH (60 % suspension in mineral oil, 13.6 g, 0.34 mol). After a clear solution was obtained (ca. 1 h), 58.2 g (0.34 mol) of benzyl bromide in 50 mL of THF was added dropwise in 1 h. Afterwards, the reaction mixture was allowed to reach room temperature, stirred for ca. 15 h and then hydrolyzed with an aqueous solution of NH₄Cl (16 g in 200 mL of water) to give two phases. The organic phase was separated and set aside. The aqueous layer was washed three times with ethyl ether. After the washings were poured into the organic phase, the resulting solution was treated with sodium sulfate to remove water and pumped to dryness. The oily residue was distilled under reduced pressure to give 91 g of (PhCH₂)₂C(CO₂Et)₂ (93% yield); b.p.: 190 °C (0.1 torr). ¹H NMR (CDCl₃, 20 °C, 200.13 MHz): d 7.4-7.1 (m, 10 H, Ph), 4.12 (q, 4 H, J(HH) = 7.2 Hz, CH₂CH₃), 3.24 (s, 4 H, CH₂Ph), 1.17 (t, 6 H, J(HH) = 7.2 Hz, CH₂CH₃).

### Synthesis of (PhCH₂)₂C(CH₂OH)₂ (2,2-bis(benzyl)-1,3-propandiol.

A solution of (PhCH₂)₂C(CO₂Et)₂ (30 g, 0.09 mol) in ethyl ether (150 mL) was poured by means of a dropping funnel into a suspension of LiAlH₄ (10 g) in 150 mL of ethyl ether at 0 °C under an inert atmosphere with vigorous stirring. The resulting reaction mixture was then allowed to warm to room temperature, refluxed for 2 h, cooled again to 0 °C and, finally, poured slowly into an aqueous solution of HCl (3.4 M) maintained at 0 °C. The mixture was transferred to a separatory funnel. The ether phase was separated, washed several times with distilled water and then treated with sodium sulfate to remove water. Upon removal of the solvent under reduced pressure, (PhCH₂)₂C(CH₂OH)₂ was obtained as an off-white solid (20.3 g, 90 % yield). ¹H NMR (CDCl₃, 20 °C, 200.13 MHz): d 7.3-7.1 (m, 10 H, Ph), 3.57 (d, 4 H, J(HH) = 5.2 Hz, CH₂OH), 2.76 (s, 4 H, CH₂Ph), 1.93 (t, 2 H, J(HH) = 5.2 Hz, CH₂OH).

### Synthesis of (PhCH₂)₂C(CH₂Cl)₂ (2,2-bis(benzyl)-1,3-dichlorogropane.

To a solution of (PhCH₂)₂C(CH₂OH)₂ (14.4 g, 0.06 mol) in 40 mL of anhydrous pyridine at 0 °C was added with stirring under an inert atmosphere 16 mL (26 g, 0.21 mol) of SOCl₂ in ca. 2 h. The resulting mixture was refluxed for 2 h and then stirred at room temperature for 15 h. After cooling to 0 °C, equal volumes of CH₂Cl₂ and water (200 mL each) were added to the reaction mixture. The separated organic layer was washed with an aqueous solution of HCl (pH 4) and then with distilled water. After the CH₂Cl₂ solution was treated with sodium sulfate, the solvent was removed under reduced pressure. To the resulting oily residue was added ethyl ether (30 mL) until an off-white solid precipitated, which was recrystallized from ethanol to give 10 g of crystalline (PhCH₂)₂C(CH₂Cl)₂ (61% yield). ¹H NMR (CDCl₃, 20 °C, 200.13 MHz): d 7.5-7.2 (m, 10 H, Ph), 3.23 (s, 4 H, CH₂Cl), 2.91 (s, 4 H, CH₂Ph).

### Synthesis of (NaO₃S(C₆H₄)CH₂)₂C(CH₂Cl)₂ (2,2-bis(p-Na-sulfonato-benzyl)-1,3-dichloropropane.

A suspension of (PhCH₂)₂C(CH₂Cl)₂ (5.6 g, 19 mmol) in 25 mL of 96 % H₂SO₄ was stirred at 100 °C for 3 h under inert atmosphere, then for 15 h at room temperature. To the resulting mixture, cooled to 0 °C, were added cold water (200 mL) and then a cold aqueous 20 % solution of NaOH until a neutral, brown solution was obtained. This was concentrated under reduced pressure to 100 mL. Upon addition of MeOH (40 mL), sodium sulfate precipitated. All the salt material was filtered off after 15 h. The remaining solution was evaporated under reduced pressure to give an oily residue which transformed into off-white crystals of (NaO₃S(C₆H₄)CH₂)₂C(CH₂Cl)₂ upon addition of ethanol (30 mL). Yield 4 g, 42 %. ¹H NMR (CDCl₃, 20 °C, 200.13 MHz): d 7.69 (d, 4 H, J(HH) = 8.3 Hz, o-(C₆H₄)SO₃Na), 7.37 (d, 4 H, J(HH) = 8.3 Hz, m-(C₆H₄)SO₃Na), 3.14 (s, 4 H, CH₂Cl), 2.86 (s, 4 H, (C₆H₄)CH₂).

### Synthesis of (NaO₃S(C₆H₄)CH₂)₂C(CH₂PPh₂)₂ (2,2-bis(p-Na-sulfonato-benzyl)-1,3-diphenyphosphinopropane.

To a solution of KPPh₂, obtained by reacting KOBu^{t} (2.3 g, 20.6 mmol) with a solution of HPPh₂ (3.8 g, 20.6 mmol) in 50 mL of dimethyl sulfoxide at room temperature under an inert atmosphere, was added dropwise 30 mL of a solution of (NaO₃S(C₆H₄)CH₂)₂C(CH₂Cl)₂ (4.3 g, 8.65 mmol) in dimethy sulfoxide. After the resulting orange solution was heated at 100 °C for 5 h, the solvent was removed under reduced pressure to give a yellow oily residue. Treatment of this latter with a saturated solution of NaCl (100 mL) under vigorous stirring gave (NaO₃S(C₆H₄)CH₂)₂C(CH₂PPh₂)₂ as an off-white solid which was recrystallized from a 1:1 mixture (w) of methanol/ethanol; 6 g, 87 % yield. ³¹P{¹H} NMR (MeOH-d₄, 20 °C, 81.01 MHz): d 27.2 (s). ¹H NMR (MeOH-d₄, 20 °C, 200.13 MHz) d 7.55 (d, 4 H, J(HH) = 8.2 Hz, o-(C₆H₄)SO₃Na), 7.3-7.1 (m, 20 H, Ph), 7.14 (d, 4 H, m-(C₆H₄)SO₃Na), 2.89 (s, 4 H, (C₆H₄)CH₂), 1.93 (br d, 4 H, J(HP) = 2.7 Hz, CH₂PPh₂). Through procedures which are analogous to that described above for (NaO₃S(C₆H₄)CH₂)₂C(CH₂PPh₂)₂ (or through alternative, ordinary organic reactions) using appropriate reagents, the following products were similarly obtained:
(MO₃S(C₆H₄)CH₂)₂C(CH₂YR¹R²)₂ where Y = P, As; R¹, R² = CH₃, C₂H₅, ^{t}-C₄H₉, C₆H₁₁, C₆H₅; M = H, Li, Na, K.
MO₃S(C₆H₄)CH₂CRC(CH₂YR¹R²)₂ where Y = P, As; R = H, CH₃; R¹, R² = CH₃, C₂H₅, ^{t}-C₄H₉, C₆H₁₁, C₆H₅; M = H, Li, Na, K.

The ligands described above were employed for the preparation of metal complexes, either in situ or isolated, with transition metals, particularly with Pd, Rh, Ir, Ru, Pt, Ni, Co in various oxidation states and coordination numbers. These complexes have been employed directly or after their anchoring on appropriate inorganic and organic supports, as catalysts in various known reactions in aqueous media, in liquid-biphase systems (in which one phase is water), or in heterogeneous systems (liquid-solid). Some examples are given below:

### Alternate copolymerization of CO and ethene in water.

The use of catalytic amounts of either

Na₂[{(O₃S(C₆H₄)CH₂)₂C(CH₂PPh₂)₂}Pd(O₂CCR₃)₂] (R = H, F) or of (NaO₃S(C₆H₄)CH₂)₂C(CH₂PPh₂)₂ and Pd(O₂CCR₃)₂ (R = H, F) in the presence of a 200-fold excess of trifluoroacetic acid (acid to catalyst ratio = 200) gave the polyketone copolymer with a productivity of 7.8 Kg of copolymer per g of Pd (68 bar, 1:1 carbon monoxide/ethene; 85 °C).

### Terpolymerization of CO, ethene and propene in water.

The use of catalytic amounts of either Na₂[{(O₃S(C₆H₄)CH₂)₂C(CH₂PPh₂)₂}Pd(O₂CCR₃)₂] (R = H, F) or of (NaO₃S(C₆H₄)CH₂)₂C(CH₂PPh₂)₂ and Pd(O₂CCR₃)₂] (R = H, F) in the presence of a 200-fold excess of trifluoroacetic acid (acid to catalyst ratio = 200) gave the terpolymer with a productivity of 4.9 Kg of terpolymer per g of Pd (68 bar, 1:1 carbon monoxide/ethene;10 g liquid propene ; 85 °C).

### Hydroformylation of alkenes in liquid-biphase systems.

The hydroformylation of 1-hexene was carried out in water/*i*-octane with a 1:1 mixture of CO and H₂ (30 bar) in the presence of catalytic amounts of Na[{(O₃S(C₆H₄)CH₂)₂C(CH₂PPh₂)₂}Rh(H₂O)₂] with the following results: total conversion 95.3 %, aldehydes 59.8 %, iso/n = 1.4 (substrate:catalyst = 100; 5 h, 80°C).

### Reduction of ketones to alcohols in liquid-biphase systems.

The hydrogenation (15 atm H₂) of trans-PhCH=CH(COMe) was carried out in water/*n*-hexane in the presence of catalytic amounts of (NaO₃S(C₆H₄)CH₂)₂C(CH₂PPh₂)₂ and RuCl₃·6H₂O with the following results: PhCH₂CH₂(COMe) 88 %, PhCH₂CH₂CH(OH)Me (12 %) (substrate:catalyst = 100; 5 h; 100°C).

### Reduction of alkenes in heterogeneous systems.

After anchoring the complex Na[{(O₃S(C₆H₄)CH₂)₂C(CH₂PPh₂)₂}Rh(H₂O)₂] over activated SiO₂ (Davinson, ca. 200 m²/g) using standard procedures, a catalytic amount of the resulting solid was used in a hydrogenation reaction of styrene which was totally converted to ethylbenzene after 2 h (substrate:catalyst = 100; 30 bar H₂; 100°C).

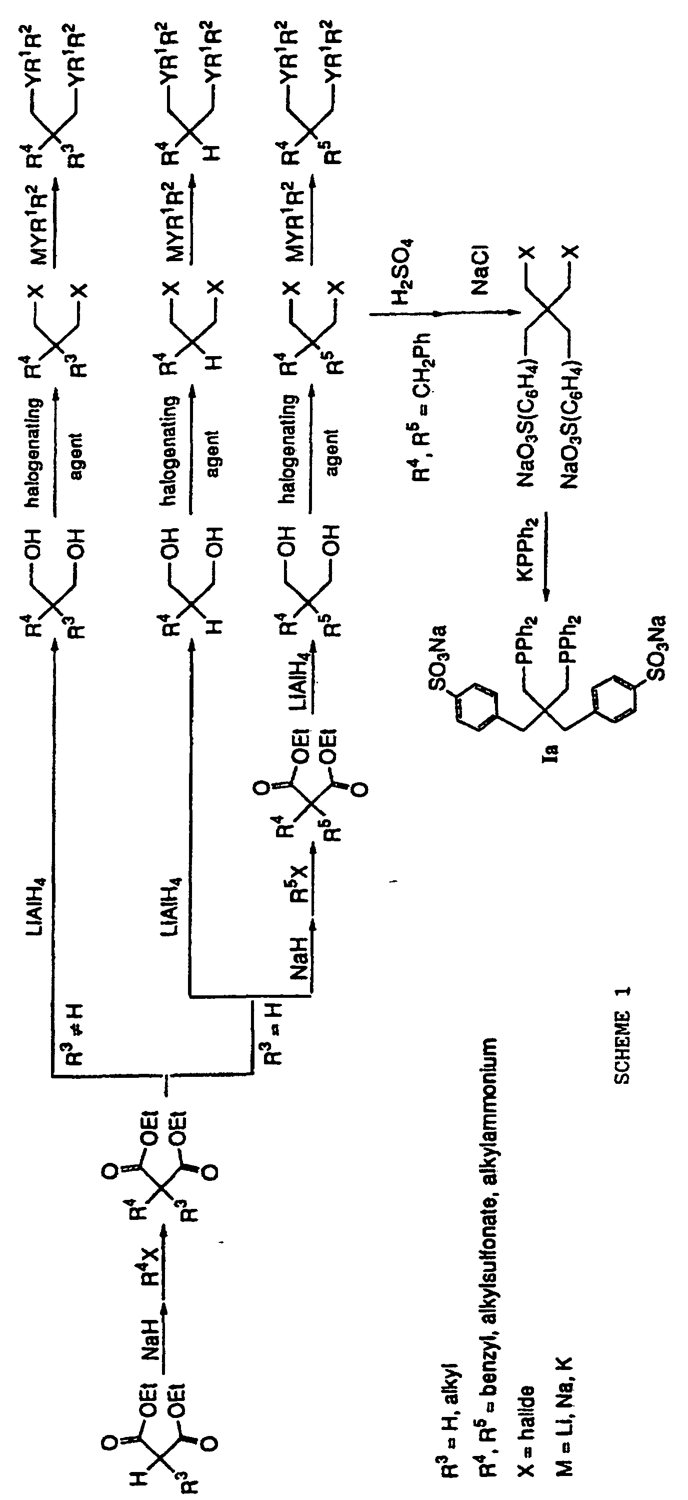

## Claims

1. Compounds of formula (I) 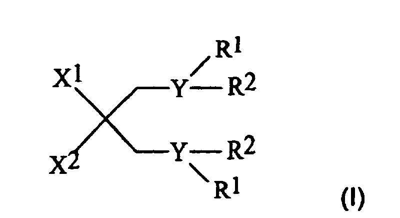 wherein R¹ and R² represent hydrocarbyl groups, identical or different from each other, X¹ is chosen from the group consisting of: hydrogen atom, a hydrocarbyl group or a polar group, X² represents a polar group, Y is chosen from the group consisting of phosphorus and arsenic, wherein such polar group is chosen in the group consisting of: aryl- or alkyl sulfonic acid, sulfonate alkali salt (-SO₃H, - SO₃⁻M⁺ wherein M⁺ is Li, Na, K) and a quaternary ammonium salt.

2. Compounds as claimed in claim 1 **characterized in that** the hydrocarbyl group is a linear, branched or cyclic alkyl group containing from 1 to 6 carbon atoms or an aryl group possibly alkyl-substituted.

3. Compounds as claimed in claim 2 as indicated below:
(MO₃S(C₆H₄)CH₂)₂C(CH₂YR¹R²)₂ wherein Y = P, As,; R¹, R² = aryl, linear, branched or cyclic alkyl containing up to 6 carbon atoms; M = H, Li, Na, K.
MO₃S(C₆H₄)CH₂CR(CH₂YR¹R²)₂ wherein Y = P, As; R = H, Me; R¹, R² = aryl, linear, branched or cyclic alkyl containing up to 6 carbon atoms; M = H, Li, Na, K.

4. Use of the compounds of the general formula (I) as claimed in claim 1 for the preparation of complexes with transition metals.

5. Complexes consisting of a transition metal and at least one compound as claimed in claim 1.

6. Complexes, isolated or generated in solution, as claimed in claim 5 **characterized in that** the transition metal is chosen in the group consisting of: Pd, Rh, Ir, Ru, Pt, Ni, Co in various oxidation states and coordination numbers.

7. Complexes as claimed in claim 5 indicated as follows: Na₂[{(O₃S(C₆H₄)CH₂)₂C(CH₂PPh₂)₂}Pd(O₂CCR₃)₂] (R = H, F), Na[{(O₃S(C₆H₄)CH₂)₂C(CH₂PPh₂)₂}Rh(H₂O)₂],
Na[{(O₃S(C₆H₄)CH₂)₂C(CH₂PPh₂)₂}Rh(COD)] (COD = 1,5-cyctooctadiene), Na[{(O₃S(C₆H₄)CH₂)₂C(CH₂PPh₂)₂}Ir(COD)],
[{(O₃S(C₆H₄)CH₂)₂C(CH₂PPh₂)₂}₂Ru(H₂O)₂].

8. Process for the preparation of the compounds as claimed in claim 1 **characterized in that** the preparation is carried out by the reaction between the 1,3-dihalopropane product substituted in the 2-position with one or more polar groups and bis-organyl-substituted phosphide or arsenide alkali salt.

9. Use of the complexes as claimed in claim 6 either as catalysts in reactions carried out in aqueous media or polyphasic systems in which the polar phase is water, or as heterogeneous catalysts (liquid-solid reactions) after the metal complexes have been anchored to appropriate inorganic or organic supports.

10. Use as claimed in claim 9 of the complex Na₂[{(O₃S(C₆H₄)CH₂)₂C(CH₂PPh₂)₂}Pd(O₂CCR₃)₂] (R = H, F) in reactions of copolymerization of CO/alkenes, terpolymerization of CO/ethene/alkenes, telomerization and hydrodimerization of alkenes and dienes, metathesis of alkenes, reactions of alkenes with alkyl and aryl halides and pseudohalides (Heck reactions).

11. Use of the complex Na[{(O₃S(C₆H₄)CH₂)₂C(CH₂PPh₂)₂}Rh(H₂O)₂] in hydrogenation and hydroformylation reactions of alkenes.

12. Use of the complex Na[{(O₃S(C₆H₄)CH₂)₂C(CH₂PPh₂)₂}Rh(COD)] (COD = 1,5-cyclooctadiene) in hydrogenation and hydroformylation reactions of alkenes.

13. Use of the complex Na[{(O₃S(C₆H₄)CH₂)₂C(CH₂PPh₂)₂}Ir(COD)] in reactions of reduction of saturated and unsaturated ketones.

14. Use of the complex [{(O₃S(C₆H₄)CH₂)₂C(CH₂PPh₂)₂}₂Ru(H₂O)₂] in the following reactions: reduction of ketones, of imines, of enamines, of nitriles, and of nitrogen heterocycles (pyridines, quinolines, pyrazoles, carbazoles).

## Patentansprüche

1. Verbindungen der Formel (I) 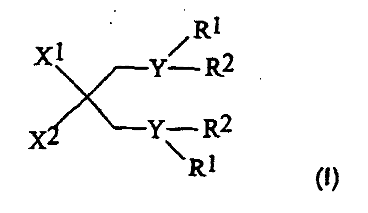 wobei R¹ und R² Kohlenwasserstoffgruppen darstellen, die identisch oder voneinander verschieden sind, X¹ aus der Gruppe ausgewählt wird, die aus einem Wasserstoffatom, einer Kohlenwasserstoffgruppe oder einer polaren Gruppe besteht, X² eine polare Gruppe darstellt, Y aus der Gruppe ausgewählt wird, die aus Phosphor und Arsen besteht, wobei eine solche polare Gruppe ausgewählt ist aus der Gruppe bestehend aus Aryl- oder Alkylsulfonsäure, Sulfonatalkalisalz(-SO₃ H, -SO₃⁻M⁺ wobei M⁺ Li, Na, K ist) und einem quaternären Ammoniumsalz.

2. Verbindungen nach Anspruch 1, **dadurch gekennzeichnet, dass** die Kohlenwasserstoffgruppe eine lineare, verzweigte oder zyklische Alkylgruppe, die 1 bis 6 Kohlenstoffatome oder eine Arylgruppe beinhaltet, die möglicherweise alkylsubstituiert ist.

3. Verbindungen nach Anspruch 2 wie unten angegeben: (MO₃S(C₆H₄)CH₂)₂C(CH₂YR¹R²)₂, wobei Y=P, As; R¹, R² = Aryl, lineares, verzweigtes oder zyklisches Alkyl, enhaltend bis zu 6 Kohlenstoffatomen; M=H, Li, Na, K ist; MO₃S(C₆H₄)CH₂CR(CH₂YR¹R²)₂, wobei Y=P, As; R=H, Me; R¹, R²=Aryl, lineares, verzweigtes oder zyklisches Alkyl, enthaltend bis zu 6 Kohlenstoffatomen; M=H, Li, Na, K ist.

4. Verwendung der Verbindungen der allgemeinen Formel (I) wie nach Anspruch 1 für die Herstellung von Komplexen mit Übergangsmetallen.

5. Komplexe bestehend aus einem Übergangsmetall und wenigstens einer Verbindung nach Anspruch 1.

6. Komplexe; isoliert oder in einer Lösung erzeugt, wie in Anspruch 5 beansprucht, **dadurch gekennzeichnet, dass** das Übergangsmetall ausgewählt wird aus der Gruppe bestehend aus Pd, Rh, Ir, Ru, Pt, Ni, Co in verschiedenen Oxidationszuständen und Koordinationszahlen.

7. Komplexe nach Anspruch 5 und wie folgt angegeben:
Na₂[{(O₃S(C₆H₄)CH₂)₂C(CH₂PPh₂)₂}Pd(O₂CCR₃)₂] (R=H, F); Na[{(O₃S(C₆H₄)CH₂)₂C(CH₂PPh₂)₂}Rh(H₂O)₂],
Na [{(O₃S(C₆H₄)CH₂)₂C(CH₂PPh₂)₂}Rh(COD)] (COD=1, 5-Zyklooctadien), Na[{(O₃S(C₆H₄)CH₂)₂C(CH₂PPh₂)₂}Ir(COD)], [{(O₃S(C₆H₄)CH₂)₂C(CH₂PPh₂)₂}₂Ru(H₂O)₂].

8. Verfahren zur Herstellung der Verbindungen nach Anspruch 1, **dadurch gekennzeichnet, dass** die Herstellung ausgeführt wird durch die Reaktion zwischen dem 1,3-Dihalopropan-Produkt, das in der Position 2 substituiert ist mit einer oder mehreren polaren Gruppen, und bisorganyl substituiertem Phosphid- oder Arsenidalkalisalz.

9. Verwendung der Komplexe nach Anspruch 6, entweder als Katalysatoren in Reaktionen, die in wässrigen Medien oder in polyphasigen Systemen, in welchen die polare Phase Wasser ist, durchgeführt werden, oder als heterogene Katalysatoren (flüssig-fest Reaktionen) nachdem die Metallkomplexe an geeigneten anorganischen oder organischen Trägern verankert worden sind.

10. Verwendung nach Anspruch 9 Na₂[{(O₃S(C₆H₄)CH₂)₂C(CH₂PPh₂)₂}Pd(O₂CCR₃)₂] (R=H, F) in Reaktionen von Copolymerisation von CO/Alkenen, Terpolymerisation von CO/Ethen/Alkenen, Telomerisation und Hydrodimerisation von Alkenen und Dienen, Metathese von Alkenen, Reaktionen von Alkenen mit Alkyl und Arylhalogeniden und Pseudohalogeniden (Heck Reaktionen).

11. Verwendung des Komplexes
Na [{(O₃S(C₆H₄)CH₂)₂C(CH₂PPh₂)₂}Rh(H₂O)₂] in Hydrierung und Hydroformylierungsreaktionen von Alkenen.

12. Verwendung des Komplexes Na[{(O₃S(C₆H₄)CH₂)₂C(CH₂PPh₂)₂}Rh(COD)] (COD=1,5 Cyclooctadien) in Hydrierungs- und Hydroformylierungsreaktionen von Alkenen.

13. Verwendung des Komplexes Na[{(O₃S(C₆H₄)CH₂)₂C(CH₂PPh₂)₂}Ir(COD)] in Reduktionsreaktionen von gesättigten und ungesättigten Ketonen.

14. Verwendung des Komplexes [{(O₃S(C₆H₄)CH₂)₂C(CH₂PPh₂)₂}₂Ru(H₂O)₂] in den nachfolgenden Reaktionen: Reduktion von Ketonen, Iminen, Enaminen , Nitrilen und von Stickstoffheterozyklen (Pyridine, Chinoline, Pyrazole, Carbazole).

## Revendications

1. Composés de formule (I) 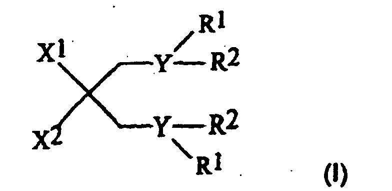 où R¹ et R² représentent des groupes hydrocarbyles, identiques ou différents l'un de l'autre, X¹ est choisi dans le groupe consistant en : atome d'hydrogène, un groupe hydrocarbyle ou un groupe polaire, X² représente un groupe polaire, Y est choisi dans le groupe consistant en phosphore et arsenic, où un tel groupe polaire est choisi dans le groupe consistant en acide aryl- ou alkyl sulfonique, sel alcalin de sulfonate (-SO₃H, -SO₃⁻M⁺ où M⁺ est Li, Na, K) et un sel d'ammonium quaternaire.

2. Composés selon la revendication 1 **caractérisés en ce que** le groupe hydrocarbyle est un groupe alkyle linéaire, ramifié ou cyclique contenant de 1 à 6 atomes de carbone ou un groupe aryle éventuellement alkyle substitué.

3. Composés selon la revendication 2 comme indiqués ci-dessous :
(MO₃S(C₆H₄)CH₂)₂C(CH₂YR¹R²)₂ où Y=P, As; R¹, R²=aryle, alkyle linéaire, ramifié ou cyclique contenant jusqu'à 6 atomes de carbone ; M = H, Li, Na, K.
(MO₃S(C₆H₄)CH₂CR(CH₂YR¹R²)₂ où Y = P, As; R = H, Me ; R¹, R² = aryle, alkyle linéaire, ramifié ou cyclique contenant jusqu'à 6 atomes de carbone ; M = H, Li, Na, K.

4. Utilisation des composés de la formule générale (I) selon la revendication 1 pour la préparation de complexes avec des métaux de transition.

5. Complexes consistant en un métal de transition et au moins un composé tel que revendiqué à la revendication 1.

6. Complexes, isolés ou générés en solution, selon la revendication 5, **caractérisés en ce que** le métal de transition est choisi dans le groupe consistant en : Pd, Rh, Ir, Ru, Pt, Ni, Co à divers états d'oxydation et nombres de coordination.

7. Complexes selon la revendication 5 indiqués comme suit :
Na₂[{(O₃S(C₆H₄)CH₂)₂C(CH₂PPh₂)₂}Pd(O₂CCR₃)₂] (R = H, F),
Na [{(O₃S(C₆H₄)CH₂)₂C(CH₂PPh₂)₂}Rh(H₂O)₂],
Na [{(O₃S(C₆H₄)CH₂)₂C(CH₂PPh₂)₂)Rh(COD)] (COD = 1,5-cyclooctadiène),
Na[{(O₃S(C₆H₄)CH₂)₂C(CH₂PPh₂)₂}Ir(COD)],
[{(O₃S(C₆H₄)CH₂)₂C(CH₂PPh₂)₂}₂Ru(H₂O)₂].

8. Procédé pour la préparation des composés tels que revendiqués à la revendication 1, **caractérisé en ce que** la préparation est effectuée par la réaction entre le 1,3-dihalopropane produit substitué à la position 2 avec un ou plusieurs groupes polaires et sel alcalin d'arseniure ou de phosphure bis-organyle substitué.

9. Utilisation des complexes selon la revendication 6 soit comme catalyseurs dans des réactions effectuées dans des milieux acqueux ou des systèmes polyphasés où la phase polaire est l'eau, ou en tant que catalyseurs hétérogènes (réactions liquide-solide) après que les complexes de métal ont été ancrés à des supports organiques ou inorganiques appropriés.

10. Utilisation selon la revendication 9 du complexe Na₂[{(O₃S(C₆H₄)CH₂)₂C(CH₂PPh₂)₂}Pd(O₂CCR₃)₂] (R = H, F), dans des réactions de copolymérisation de CO/alcènes, terpolimérisation de CO/éthène/alcènes, télomérisation et hydrodimérisation d'alcènes et diènes, métathèse d'alcènes, réactions d'alcènes avec des halogénures et pseudohalogénures d'alkyle et d'aryle (réactions de Heck).

11. Utilisation du complexe Na[{(O₃S(C₆H₄)CH₂)₂C(CH₂PPh₂)₂}Rh(H₂O)₂] dans les réactions d'hydrogénation et d'hydroformylation des alcènes.

12. Utilisation du complexe Na[{(O₃S(C₆H₄)CH₂)₂C(CH₂PPh₂)₂}Rh(COD)] (COD = 1,5-cyclooctadiène) dans les réactions d'hydrogénation et d'hydroformylation des alcènes.

13. Utilisation du complexe Na [{(O₃S(C₆H₄)CH₂)₂C(CH₂PPh₂)₂}Ir(COD)] dans les réactions de réduction de cétones saturées et insaturées.

14. Utilisation du complexe [{(O₃S(C₆H₄)CH₂)₂C(CH₂PPh₂)₂}₂Ru(H₂O)₂] dans les réactions suivantes : réduction des cétones, des imines, des énamines, des nitriles et des hétérocycles d'azote (pyridines, quinoléines, pyrazoles, carbazoles).
